# EUROPEAN PATENT APPLICATION

(11) **EP 3 556 342 A1**
(43) Date of publication of application: **23.10.2019**
(21) Application number: 18382258.4
(22) Date of filing: 17.04.2018
(51) Int. Cl.: A61K 8/25, A61K 8/44, A61K 8/49, A61K 8/64, A61Q 19/06

(54) **COSMETIC MULTILEVEL METHOD FOR CELLULITE AND/OR SKIN FLACCIDITY**

(71) Applicant: Acpac Biotech, S.L., 08921 Santa Coloma de Gramenet (ES)
(72) Inventor: PINTO, HERNÁN RAÚL, 08921 SANTA COLOMA DE GRAMENET (ES)
(74) Representative: ZBM Patents - Zea, Barlocci & Markvardsen

(57) **Abstract**

Present invention relates to a cosmetic method for ameliorating the unaesthetic appearance of skin caused by a condition selected from skin flaccidity, a lipodystrophy, and a combination thereof. The invention also relates to particular topical skin tightening compositions, as well as to cosmetic compositions for intradermal and subcutaneous administration.

## Description

### Technical Field

Present invention relates to cosmetic methods for reducing unaesthetic appearance of skin due to cellulite, local adiposities and/or skin sagging or flaccidity.

### Background Art

It is widely known that for many and varied reasons appearance of skin is challenged along life. Without being any pathology, lipodystrophies including local adiposities as well as cellulite, usually in combination with skin sagging give a physical aspect not well-accepted by people suffering them and that in many occasions evolves to a certain social impact undermining self-confidence.

Cellulite, medically known as edematous-fibrosclerotic panniculopathy, is a common condition in women. It is catalogued as a lipodystrophy. Cellulite does not represent any risk for the health, but it is an unaesthetic condition. Its ethology seems a complex combination of a myriad of factors including hormone unbalancing, genetic predisposition, as well as inflammation and lifestyle. It is manifested by an orange-peel or dimpled appearance of the skin and is commonly developed in the tight, buttock and hips regions. Cellulite develops in the hypodermis or subcutaneous fat layer, being fat lobes surrounded by connective tissue. When this connective tissue become weak, the main cell types in the subcutaneous tissue, in particular adipocytes bulged up and cause the dimpled appearance of skin. Cellulite is not related with obesity or overweight, since thin women do also have cellulite.

Another type of lipodystrophy is called localized adiposity, in which adipose tissue is not homogeneously distributed in subcutaneous tissue, but regionally or circumscribed in certain regions. In biology, adipose tissue, body fat or simply fat is a loose connective tissue composed mostly of adipocytes. In addition to adipocytes, adipose tissue contains the stromal vascular fraction (SVF) of cells including preadipocytes, fibroblasts, vascular endothelial cells and a variety of immune cells such as adipose tissue macrophages.

Finally, skin sagging also known as skin flaccidity or even excess skin is the loss of elasticity and firmness of skin that results in a generalized sagging effect of skin. The main causes of skin sagging are mechanical, for example due to a high skin expansion during pregnancy or due to a fast weight gain and loss. Nonetheless, main cause of the loss of elasticity and skin firmness is the progressive functional impairment of dermis due to skin aging.

Many have been the compositions (mainly topical or oral) and methods to treat any of the above listed conditions, sometimes in combination with special diets, massages and lymph drainages that have not had the expected results. Among the methods for facing cellulite highly invasive techniques have been employed, such as liposuction, which consists mainly in partially aspirating subcutaneous fat. This technique involves making several small incisions and the suction of fat with cannulae. However, beside the inherent risks of being a surgical invasive treatment, liposuction may lead to unattractive results if not properly performed.

Combined cosmetic methodologies are being employed nowadays. Some of them are discussed for example in the document of Alster et al., "Treatment of cellulite with optical devices: an overview with practical considerations", Lasers in Surgery and Medicine 2006, vol. 38, pp:727-730, wherein skin regions were submitted to radiofrequency, infrared light and mechanical suction with a device known as Velasmooth®. This document also mentions the use of combined diode laser energy with tissue massage.

Also patent application with publication number WO2013093947 (MOTOLESE Pasquale) discloses a cosmetic injectable composition to reduce cellulite, said composition comprising an iron ion chelating agent, an osmotic diuretic, amino acids and vitamins.

Moreover, two products for reducing cellulite are nowadays marketed as Alidya® and Aqualix®. Alidya® is a formulation comprising amino acids, α 1-4 glycoside, and EDTA designed to solubilize and remove toxic extracellular elements; for extracellular matrix alkalinisation, to improve tissue oxygenation and to provide the constituents for matrix restructuring and to normalize adipocyte function of the subcutaneous adipose tissue. The composition is injected by mesotherapy, thus intradermally, in the cellulite area in weekly sessions. On the other hand, Aqualix® is an injectable composition comprising detergents in a sugar matrix with adipocytolytic effect. It is used for localized adiposity that is administered using intralipotherapy.

Other examples of cosmetic methods for the removal of localized fat are disclosed in the patent document EP1928466 (Biolitec Unternehmensbeteiligungs II AG). EP1928466 proposes and shows the administration of a photosensitive agent (Temoporfin) that is locally injected by mesotherapy, into the cellulite area to be treated and then it is activated using light. This document proposes using also the photosensitive agent in combination lymph drainages and gel application. Several approaches are used depending on the cellulite areas (depressed areas, elevated zones, etc.). Although effective, it is a complex method requiring complex instrumentation and in some cases several days for reactivating the photosensitive agent.

The patent document EP0857480 (Régie Municipale des Eaux Minérales de Royat) discloses using a particular thermal gas for the cosmetic treatment of cellulite, said gas being injected into the hypodermis in the area of the cellulite mass. Particular schedules of treatment in several days are also disclosed, as well as the combination with other cosmetic methods for cellulite.

On the other side, in EP2263683 (Lavo Juversa Co) the use of basic fibroblast growth factor (bFGF) is disclosed for a cosmetic treatment of aging of skin, in particular for skin sagging, skin wrinkles and rough skin. The treatment comprises the administration of a cosmetically effective amount of bFGF intradermally or subcutaneously.

Albeit the efforts for ameliorating skin appearance when cellulite, local adiposities and skin sagging is present, there is still a need of alternative cosmetic compositions and cosmetic methods to improve skin appearance.

### Summary of Invention

A first aspect of the invention is a cosmetic method for ameliorating the unaesthetic appearance of skin caused by a condition selected from skin flaccidity, a lipodystrophy and a combination thereof, the method comprising topically administering a cosmetic skin tightening composition, which is the same of a cosmetic composition capable of skin tightening, in combination with two cosmetic compositions capable of promoting collagen synthesis in a zone or area of the skin with the unaesthetic condition, said compositions capable of promoting collagen synthesis being administered at different depths of the anatomic structure defined by the skin and the subcutaneous tissue.

Inventors have realised that the combination of administering several compositions applied or directed to different levels or depths in anatomic structure defined by skin structure (epidermis, dermis) and the subcutaneous tissue imply effective results in cellulite and skin sagging cases.

Without being bound to any theory, inventors postulate that active ingredients able to promote collagen synthesis, administered in combination to an area with lipodystrophy (cellulite or localized adiposities) and/or an area with skin flaccidity, do act synergistically when applied to different levels and they allow a better amelioration of skin appearance in comparison with isolated treatments. They also allow a fast way to ameliorate the said unaesthetic condition. Thus, it is promoted the synthesis and/or maintenance of collagen and other extracellular matrix proteins by different ways and at different skin levels. Thus, any of the faced conditions are treated by different combined approaches that synergistically ameliorate skin appearance. In other words, the cosmetic method is a multilevel cosmetic method for ameliorating skin appearance. Promotion of the synthesis and/or maintenance of collagen and other extracellular matrix proteins is translated to an increase of skin elasticity and increase of skin firmness. Thus, the two cosmetic compositions capable of promoting collagen synthesis are, indeed, cosmetic compositions comprising, among other compounds, peptides from 3 to 50 amino acids length and capable to increase skin elasticity and skin firmness to an area with lipodystrophy and/or an area with skin flaccidity. The peptides are in particular, ones categorized as skin conditioning peptides according to INCI classification, and they comprise amino acids with bulky side chains and selected from proline, hydroxyproline, methionine, tyrosine, cysteine, leucine, isoleucine, and also charged side chains selected from arginine, aspartic and glutamic. The increase is to be understood in relation with initial elasticity or skin firmness or with a non-treated area.

The cosmetic method of the invention does not imply any health risk as will be depicted below. Thus, it does not imply any complication that would be of health impact. The cosmetic method is a non-therapeutical method for ameliorating the unaesthetic appearance of skin caused by a condition selected from skin flaccidity, a lipodystrophy and a combination thereof.

This first aspect can be re-worded in different formats. Thus, the invention is also related with the use of a topical skin tightening composition in combination with at least two cosmetic compositions capable of promoting collagen synthesis and for being administered at different depths of skin structure, for ameliorating the unaesthetic appearance of skin caused by a condition selected from skin flaccidity, a lipodystrophy and a combination thereof.

In a second aspect, the invention also relates to kits for ameliorating the unaesthetic appearance of skin caused by a condition selected from skin flaccidity, a lipodystrophy and a combination thereof, said kit comprising:
- a topical skin tightening composition;
- two skin cosmetic compositions capable of promoting collagen synthesis and for being administered at different depths of the anatomic structure defined by the skin and the subcutaneous tissue;
- optionally, means for intradermal and subcutaneous administration of the compositions for promoting collagen synthesis; and
- optionally, use instructions.

The kits of the invention provide in an adequate format all the actives to be administered in combination in one or more sessions to achieve amelioration of skin appearance.

Many different topical skin tightening compositions (in other words compositions capable of tightening the skin) could be used in the present invention. Nonetheless, inventors propose a new topical one that properly works in conjunction with the at least two cosmetic compositions for promoting collagen synthesis.

Thus, a third aspect of the invention is a topical skin tightening composition comprising a cosmetically effective amount of a sesame hydrolysed protein, a soy hydrolysed protein, organic silicium and hydroxyproline, together with cosmetically acceptable excipients or carriers.

Inventors have also surprisingly found that with a particular combination of amino acids and one or more antioxidant compounds, a cosmetic composition for subcutaneous administration could be prepared with an excellent effect in reducing skin flaccidity and/or cellulite and/or any localized adiposity, especially when is combined with a topical composition and a composition for intradermal administration, according to the method of the present invention.

Thus, in a fourth aspect the invention relates also to cosmetic compositions for subcutaneous administration cosmetically effective amounts of one or more amino acids, one or more antioxidant compounds, wherein one of the antioxidant compounds is lipoic acid, together with cosmetically acceptable excipients or carriers.

Yet in a fifth aspect, the invention relates to cosmetic compositions for intradermal administration comprising cosmetically effective amounts of one or more co-factors for the enzymatic synthesis of collagen by fibroblasts cells and one or more antioxidant compounds, together with cosmetically acceptable excipients or carriers. These compositions imply the effect of stimulate the fibroblasts in the dermis to synthesise new collagen molecules and other proteins of the extracellular matrix, such as elastin and/or fibrin, at the same time the composition provides an antioxidant environment in the area where it is administered and that allows the protection from oxidation by free radicals of the newly formed and of the old collagen in the treated area. With this, preservation of the collagen functionality is accomplished.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The expression "anatomic structure defined by the skin and the subcutaneous tissue" is to be understood as the multi-layered anatomic structure defined, from outer to inner of the body in a subject, the epidermis, the dermis and the subcutaneous tissue. Epidermis and dermis are properly the layers conforming skin (in particular human skin). On the other hand, the subcutaneous tissue does not form part of skin, but it is a tissue attaching the skin to underlying bone and muscles.

The "epidermis" is thus the outermost layer of the skin. It forms the waterproof, protective wrap over the body's surface which also serves as a barrier to infection and is made up of stratified squamous epithelium with an underlying basal lamina. The epidermis contains no blood vessels, and cells in the deepest layers are nourished almost exclusively by diffused oxygen from the surrounding air and to a far lesser degree by blood capillaries extending to the outer layers of the dermis. The main type of cells which make up the epidermis are Merkel cells, keratinocytes, with melanocytes and Langerhans cells also present. The other main part of skin is the "dermis", which is the layer of skin beneath the epidermis that consists of connective tissue (including fibroblasts, adipocytes, macrophages, mast cells and leucocytes) and cushions the body from stress and strain. The dermis is tightly connected to the epidermis by a basement membrane. It also harbors many nerve endings that provide the sense of touch and heat. It contains the hair follicles, sweat glands, sebaceous glands, apocrine glands, lymphatic vessels and blood vessels. The blood vessels in the dermis provide nourishment and waste removal from its own cells as well as from the Stratum basale of the epidermis. Finally, the "subcutaneous tissue" (also known as hypodermis as above exposed) is the tissue below the dermis of the skin. Its purpose is to attach the skin to underlying bone and muscle as well as supplying it with blood vessels and nerves. It consists of loose connective tissue, adipose tissue and elastin. The main cell types are fibroblasts, macrophages and adipocytes (subcutaneous tissue contains 50% of body fat). Fat serves as padding and insulation for the body.

"Human collagen" is the main structural protein in the extracellular space in the various connective tissues in animal bodies. As the main component of connective tissue, it is the most abundant protein in mammals, making up from 25% to 35% of the whole-body protein content. Collagen consists of amino acids wound together to form triple-helices to form of elongated fibrils. It is mostly found in fibrous tissues such as tendons, ligaments and skin. So far, 28 types of collagen have been identified and described. They main ones can be divided into several groups according to the structure they form. Fibrillar collagen includes type I, II, II, V and XI. Non-fibrillar collagen include type IX, XII, XIV, XIX, XXI, VIII, X, IV), XV, XVIII, XIII, XVII, VI, and VII. Collagen has an unusual amino acid composition and sequence. Glycine is found at almost every third residue. Proline makes up about 17% of collagen. Collagen contains two uncommon derivative amino acids not directly inserted during translation. These amino acids are found at specific locations relative to glycine and are modified post-translationally by different enzymes, both of which require vitamin C as a cofactor: Hydroxyproline derived from proline; hydroxylysine derived from lysine - depending on the type of collagen, varying numbers of hydroxylysines are glycosylated (mostly having disaccharides attached). Cortisol stimulates degradation of (skin) collagen into amino acids.

For "peptides from 3 to 50 amino acids length" is to be encompassed, according to this description, peptides categorized as skin conditioning peptides according to INCI classification, and they comprise amino acids with bulky side chains and selected from proline, hydroxyproline, methionine, tyrosine, cysteine, leucine, isoleucine, and also charged side chains selected from arginine, aspartic and glutamic. The peptides are mainly from synthetic origin or from biotechnological (microorganism) synthesis. Particular peptides are selected from commercial ones known with generic INCI and IUPAC names Oligopeptide-24, Decapeptide-4 and Tripeptide -6. Tripeptide-6 is a synthetic peptide consisting of glycine, hydroxyproline and proline. Oligopeptide-24 is the synthetic peptide containing 13 amino acids consisting of arginine, aspartic acid, cysteine, isoleucine, glutamic acid, glycine, methionine, and tyrosine. Decapeptide-4 is a synthetic peptide consisting of arginine, aspartic acid, cysteine, glutamic acid, leucine, methionine and tyrosine.

The expression "promoting collagen synthesis" is to be understood as serving to favouring, mainly in fibroblast sited in the dermis and the subcutaneous tissue, those metabolic cell routes and or steps that lead to the synthesis of collagen performed mainly by fibroblasts as well as to the synthesis of other extracellular matrix proteins, such as elastin. The synthesis is favoured by giving to the fibroblasts not only the amino acids that mainly constituted the collagen, but also the co-factors that enzymes need for such synthesis. In parallel, the synthesis is favoured by preserving existing collagen or the newly synthesised from any free radicals. All these elements allow good quality collagen be synthesised, which quality is indirectly determined by measuring elastic and mechanical features of the skin. The so-called elasticity and firmness of a particular skin in a particular area can be determined by several techniques. One of the most broadly used technologies implies the application of negative pressure to a skin area by means of an aperture into which the skin penetrates. The measure of the penetration depth and of the release time once negative pressure is stopped, are correlated with skin firmness and elasticity. These later skin properties are the result of good quality collagen and elastin synthesis, as well as of the preservation of any of them from radical oxidation in any skin layer (epidermis and dermis) and in the subcutaneous tissue. When in present invention is said that promotion of the synthesis of collagen is performed, is in particular referred to the synthesis of collagen type I, which is the main type in skin structure. Main steps in the synthesis of collagen include, besides the transcription of mRNA, the step of pre-pro-peptide formation, the step of transformation of pre-pro-peptide to pro-collagen, the step of modification in Golgi apparatus, the step of formation of tropocollagen outside the cell, and the step of formation of collagen fibril. In pre-pro-peptide formation mRNA exists from cell nucleus, enters the cytoplasm and an N-terminal signal peptide is first translated, which conducts the early peptide to endoplasmic reticulum for post-translational processing. In the step of transformation of pre-pro-peptide to pro-collagen the signal peptide is dissolved, lysines and prolines are hydroxylated (step that requires Vitamin C as a cofactor), said hydroxylysines are glycosylated by adding either glucose or galactose monomers, and finally three of the hydroxylated and glycosylated propeptides twist into a triple helix forming procollagen. In Golgi apparatus oligosaccharides (not monosaccharides as in step 3) are added, and then the procollagen is packaged into a secretory vesicle destined for the extracellular space. Formation of tropocollagen requires that 'collagen peptidases', remove the "loose ends" of the procollagen molecule. Finally, collagen fibril is formed by lysyl oxidase, an extracellular copper-dependent enzyme, produces the final step in the collagen synthesis pathway. This enzyme acts on lysines and hydroxylysines producing aldehyde groups, which will eventually undergo covalent bonding between tropocollagen molecules. This polymer of tropocollogen is known as a collagen fibril.

A "fibroblast" is a type of cell that synthesizes the extracellular matrix and collagen, the structural framework (stroma) for animal tissues, and plays a critical role in wound healing. Fibroblasts are the most common cells of connective tissue in animals.

The expression "co-factors for the enzymatic synthesis of collagen" relates to those vitamins and metal ions that are used by lysyl oxidase and prolyl hydroxylase. Examples of particular co-factors are vitamin C, metal ions selected from zinc, copper and combinations thereof. In general, a co-factor is a non-protein chemical compound or metallic ion that is required for an enzyme's activity. Cofactors can be considered "helper molecules" that assist in biochemical transformations. The rates at which these happen are characterized by enzyme kinetics. Organic cofactors are often vitamins or made from vitamins. Cofactors can be divided into two major groups: organic Co-factors, such as flavin or heme, and inorganic cofactors, such as the metal ions Mg2+, Cu+, Mn2+, or iron-sulfur clusters. In humans this list commonly includes iron, magnesium, manganese, cobalt, copper, zinc, and molybdenum.

For "Sesame hydrolysed protein" is to be understood any commercially available protein hydrolysate mixture of cosmetic acceptable grade and that has been prepared from any part of the sesame plant, usually from the seed. Thus, the hydrolysate comprise fragments of the plant proteins. Examples of sesame hydrolysed protein for cosmetic use include the product of Seppic known as Sesaflash™, which comprises glycerine, acrylates copolymers, polycarbamyl polyglycol ester, water and hydrolyzed sesame protein with 2-hydroxy-3-(3-(methyldihydroxysilyl)propoxy)propyl derivatives (these derivatives also termed by supplier as pg-propyl methylsilanediol).

For "Soy hydrolysed protein" is to be understood any commercially available protein hydrolysate mixture of cosmetic acceptable grade and that has been prepared from any part of the soy plant, usually from soy flour obtained by grinding soy seeds. Thus, the hydrolysate comprise fragments of the plant proteins. Examples of soy hydrolysed proteins for cosmetic use include the product of SILAB known as Raffermine®, which comprises hydrolyzed soy flour comprising glycoproteins and cellulose (a structural polysaccharide), phenoxyethanol and water.

The expression a "cosmetically acceptable", relating to any compound in this description refers to that compounds being active ingredients, excipients or carriers suitable for use in contact with human skin without undue toxicity, incompatibility, instability, allergic response, among others.

The term " cosmetically effective amounts" is defined as any amount sufficient to significantly improving the cosmetic appearance of the skin without substantial irritation, but low enough to avoid serious side effects (at a reasonable benefit/risk ratio), within the scope of sound medical judgement. The safe and effective amount of the different ingredients in the compositions administered in combination will vary with the condition of the skin, the duration of the treatment, the nature of any concurrent treatment, the specific combination of active ingredients employed, the particular cosmetically acceptable carrier utilized, and like factors in the knowledge and expertise of any attending physician.

The "INCI" name refers to the common nomenclature for ingredient labelling on the packaging of cosmetic products. The abbreviation "INCI" stands for International Nomenclature Cosmetic Ingredient. An INCI name may cover several chemical entities.

As above indicated the invention relates in a first aspect to a multilevel cosmetic method in which a safe and cosmetic effective amount of a topical composition is administered in combination with at least safe and effective amount of two compositions capable of promoting collagen synthesis and that are administered at two different depths of anatomic structure defined by the skin and the subcutaneous tissue, all with the aim of ameliorating the unaesthetic appearance of skin caused by a condition selected from skin flaccidity, a lipodystrophy and a combination thereof.

Particular embodiments of the first aspect that are enumerated below do also apply to any of the reworded possible formats previously exposed for this first aspect, as well as to particular embodiments of the other aspects.

Thus, in a particular embodiment of the cosmetic method of the invention, the compositions capable of promoting collagen synthesis and administered at two different depths of anatomic structure defined by the skin and the subcutaneous tissue are appropriate to be administered by injection at a different depth of said anatomic structure.

In a more particular embodiment of the method, one composition capable of promoting the collagen synthesis is subcutaneously administered; and one composition capable of promoting the collagen synthesis is intradermally administered. This means that the subcutaneously administered composition is applied in a depth from 6 to 7 mm in relation with the surface of skin; and the intradermally administered composition is applied in a depth from 3 to 4 mm in relation with the surface of skin.

As above indicated, in a particular embodiment they are applied by injection, more in particular by multiple injections within an area of the skin with the unaesthetic condition. More in particular, the injections are applied at several points through the area to be treated. In another particular embodiment, to squared skin areas of 10 cm x 15 cm and at intervals of 1 cm, they are applied from 50 to 60 injections of the intradermally administered composition and 9 injections of the subcutaneously administered composition. In a more particular embodiment, each injected those of the intradermally administered composition is from 0.05 cm³ to 0.1 cm³, more in particular is from 0.1 cm³. In another particular embodiment, each injected those of the subcutaneously administered composition is from 0.4 cm³ to 0.5 cm³, more in particular is from 0.5 cm³.

In another particular embodiment, optionally in combination with any embodiment above or below, one composition capable of promoting the collagen synthesis is subcutaneously administered and the subcutaneously administered composition comprises a cosmetically effective amount of amino acids, optionally one or more peptides from 3 to 50 amino acids length, and one or more antioxidant compounds together with cosmetically acceptable excipients or carriers. Amino acids are used in order the cells in hypodermis can synthesise new collagen and/or other extracellular matrix proteins, or to repair the collagen that could have been damaged. The antioxidant compound protects said new proteins and older ones from oxidation. This composition is to be understood as a composition for structuring collagen in the subcutaneous area, and thus promoting a tissue compaction of fibroblasts and adipocytes of the subcutaneous tissue.

In a more particular embodiment of the first aspect of the invention, the subcutaneously administered composition comprises amino acids selected from the group consisting of alanine, glycine, proline, leucine, lysine and combinations thereof. Combinations of amino acids is to be understood that at least two of them are present in the composition, at least three of them, at least four of them, or at least the five. More in particular, the subcutaneously administered composition comprises the five amino acids. These amino acids are selected from among D and/or L amino acids; preferably L amino acids.

Particular amounts of any of the amino acids range from 0.05 % w/w to 5.0 % w/w in the composition. In particular, the amount of alanine is from 0.2 % w/w to 2.0 % w/w in particular 0.8 %. The concentration in the composition of glycine is from 1.0 % w/w to 5.0 % w/w, in particular 1.0 %. The concentration in the composition of proline is from 0.1 % w/w to 1.0 % w/w, in particular 0.5 %. The concentration in the composition of leucine is from 0.1 % w/w to 0.5 % w/w, in particular 0.2 %. The concentration in the composition of lysine is from 0.05 % w/w to 0.3 % w/w, in particular 0.1 %.

The subcutaneously administered composition is a liquid composition, wherein all the ingredients are in a solvent or carrier, which in particular comprises water for injectables (abbreviated wfi). In some particular embodiments, other appropriate cosmetically acceptable excipients and/or carriers are added, in particular pH buffers, and preservative compounds.

Concentration of amino acids in the liquid composition range from 0.5 mg/ml to 50 mg/ml. The concentration of alanine is, in a particular embodiment, from 2 to 20 mg/ml. The concentration of glycine is, in a particular embodiment, from 10 to 50 mg/ml. The concentration of proline is, in a particular embodiment, from 1 to 10 mg/ml. The concentration of leucine is, in a particular embodiment, from 1 to 5 mg/ml. The concentration of lysine is, in a particular embodiment, from 0.5 to 3 mg/ml.

In another particular embodiment, the subcutaneously administered composition comprises peptides from 3 to 50 amino acids length. These peptides do also aim the provision of a structure that remains in the injected area in substitution of collagen. Indeed, they are ones with a composition in amino acids of human collagen. They are also provided as source of amino acids. In a more particular embodiment the peptides are peptides from 10 to 50 amino acids. More in particular are the peptides known as oligopeptide-24 and decapeptide-4. Particular amounts of the peptides range from 1x10⁻⁴ % w/w to 5 x 10⁻⁴ % w/w.

In another particular embodiment, the subcutaneously administered composition comprises lipoic acid as antioxidant compound.

In yet another particular embodiment, the subcutaneously administered composition comprises cosmetically effective amounts of hyaluronic acid or a cosmetically acceptable salt thereof. Hyaluronic acid provides also structural material to be either used by the cells in the hypodermis or to be temporarily retained in the subcutaneous area as a restructuring material in said injected area. Particular salts of hyaluronic acid are alkali metal salts, more in particular sodium salts.

In yet another particular embodiment, the subcutaneously administered composition comprises a compound selected from the nucleobases adenine, thymine, cytosine, guanine and/or uracil. In a more particular embodiment, it comprises adenine. It is to be understood that all components in a cosmetically effective amount.

In yet another particular embodiment, the subcutaneously administered composition comprises cosmetically effective amounts of vitamins selected from the group consisting of B vitamins, C vitamins, A vitamins, D vitamins, and combinations thereof. In yet a more particular embodiment, it comprises cyanocobalamin, a synthetic form if vitamin B12.

Thus, when in the present description vitamins are mentioned they refer to any vitamin from a natural source or from synthetic processes.

In a more particular embodiment the subcutaneously administered composition comprises:
- Hyaluronic acid or a salt thereof, in particular from 0.05 % to 0.5 % w/w
- Thiamine, in particular from 0.05 % to 0.5 % w/w
- Alanine, in particular from 0.2 % to 2.0 % w/w
- Cyanocobalamine, in particular from 0.001 % to 0.2 % w/w
- Glycine, in particular from 1.0 % to 5.0 % w/w
- Lipoic acid or a salt thereof, in particular from 0.005 % to 0.05 % w/w
- Proline, in particular from 0.1 % to 1.0 % w/w
- Adenine, in particular from 0.005 % to 0.2 % w/w
- Leucine, in particular from 0.1 % to 0.5 % w/w
- Lysine, in particular from 0.05 % to 0.3 % w/w
- Oligopeptide-24, in particular from 1x10⁻⁴ % w/w to 5 x 10⁻⁴ % w/w
- Decapeptide-4, in particular from 1x10⁻⁴ % w/w to 5 x 10⁻⁴ % w/w, and
- Water, amount to get 100% by weight of the composition.

In another particular embodiment of the first aspect of the invention, the intradermally administered composition is a composition capable of stimulating the collagen synthesis from fibroblasts in the dermis and for protecting collagen of free radicals.

In a more particular embodiment, one composition capable of promoting the collagen synthesis is thus intradermally administered, and the intradermally administered composition comprises a cosmetically effective amount of one or more co-factors for the enzymatic synthesis of collagen and one or more antioxidant compounds, together with cosmetically acceptable excipients or carriers.

In another more particular embodiment of the intradermally administered composition used in the method of the invention, the co-factors are selected from metal ions selected from zinc, copper and combinations thereof. In a more particular embodiment, these metal ions are provided as salts of dicarboxylic acids that are widely used in nature. More in particular are salts of gluconic acid, also known as gluconates.

In yet another particular embodiment, the antioxidant compound in the intradermally administered composition is a compound selected from the group consisting of centrophenoxine, either 4-chlorophenoxyacetic acid or a pharmaceutical and/or cosmetically acceptable ester thereof, either dimethylethanolamine (DMAE) or a pharmaceutical and/or cosmetically acceptable ester thereof, and combinations thereof. More in particular is DMAE. In another particular embodiment is centrophenoxine, which is an ester of dimethylethanolamine (DMAE) and 4-chlorophenoxyacetic acid (pCPA).

In yet another particular embodiment, the intradermal administered composition further comprises cosmetically effective amounts of one or more of a vitamin; a peptide; chondroitin sulphate or a cosmetically acceptable salt thereof, in particular a salt of an alkali metal, more in particular sodium salt; and lactic acid or a cosmetically acceptable salt thereof, in particular a salt of an alkali metal, more in particular sodium lactate. In a more particular embodiment of the first aspect, the intradermally administered composition comprises a vitamin, a peptide, sodium chondroitin sulphate and a cosmetically acceptable salt of lactic acid, in particular sodium lactate.

In even a more particular embodiment of the first aspect, the intradermal administered composition comprises ascorbic acid, sodium lactate, DMAE, zinc gluconate, copper gluconate, chondroitin sulphate, and the tripeptide-6. Therefore, as above indicated the intradermal administered composition do also comprises peptides in a particular embodiment, in particular peptides from 3 to 50 amino acids with an amino acid composition including bulky side chains amino acids and selected from proline, hydroxyproline, methionine, tyrosine, cysteine, leucine, isoleucine, and also charged side chains selected from arginine, aspartic and glutamic. More in particular, it is a tripeptide. More in particular a tripeptide comprising glycine, proline and hydroxyproline.

Chondroitin sulphate is a natural sulphated glycosaminoglycan with a polymeric structure characterized by a disaccharide with a molecular weight of 463,363 g/mol, which is repeated and formed by N-acetyl-D-galactosamine and D-glucuronic acid. Most of the N-acetyl-D-galactosamine residues are sulphated. Depending on the length of the polymeric structure the final molecular weight varies. In the present invention, chondroitin sulphate is of cosmetically grade and as accepted by Pharmacopeias. In a particular embodiment, chondroitin sulphate is obtained from enzymatic hydrolysis of shark cartilage with a controlled enzymatic hydrolysis in which excedent proteins are separated by means of ultra-filtration. Examples of chondroitin sulphates usable in the present invention are those a molecular weight comprised between 5000 daltons and 60000 daltons. These are the ones also used orally in the treatment of biological tissue reparation, reducing symptomatology of chronic venous insufficiency, and as pain reducer in arthrosis. Chondroitin sulphate usable in the composition of the invention are commercially available. Examples include sodium chondroitin sulphate (INCI name) of Fagron Iberica S.A.U. (Spain). It is usually sold as a powder, which is soluble in water at 2 % w/w.

Particular amounts of any of any of the co-factors of enzymes of the route of collagen synthesis range from 0.010 % w/w to 0.20 %w/w. In particular, the amount of dicarboxylic salt of zinc is from 0.020 % w/w to 0.20 %w/w, in particular 0.10 %. The concentration in the composition of dicarboxylic salt of copper is from 0.01 %w/w to 0.05% w/w, in particular 0.025 %.

In a particular embodiment, the intradermal administered composition is a liquid composition, wherein all the ingredients are in a solvent or carrier, which in particular comprises water for injectables. Other appropriate cosmetically acceptable excipients and/or carriers include pH buffers and/or preservative compounds. Examples of pH buffers include Tris buffer, Tris-HCI buffer and phosphate buffer saline (PBS).

Concentration of enzyme co-factors in the liquid composition range from 0.1 mg/ml to 2.0 mg/ml. The concentration of dicarboxylic salt of zinc is, in a particular embodiment, from 0.2 to 2.0 mg/ml. The concentration of dicarboxylic salt of copper is, in a particular embodiment, from 0.1 to 0.50 mg/ml.

In a more particular embodiment the intradermal administered composition comprises
- Ascorbic acid, in particular from 0.1 % to 10 % w/w
- Sodium lactate, in particular from 1.0 % to 5.0 % w/w
- DMAE, in particular from 1.0 % to 20.0 % w/w
- Zinc gluconate, in particular from 0.02 % to 0.2 % w/w
- Chondroitin sulphate, in particular from 0.1 % to 0.5 % w/w
- Copper gluconate, in particular from 0.01 % to 0.05 % w/w
- Tripeptide-6, in particular from 1x10⁻⁴ % w/w to 5 x 10⁻⁴ % w/w, and
- Water, amount to get 100% by weight of the composition.

In a particular embodiment of the first aspect of the invention, the cosmetic composition capable of skin tightening comprises a cosmetically effective amount of a sesame hydrolysed protein, a soya hydrolysed protein, organic silicium and hydroxyproline, together with cosmetically acceptable excipients or carriers, and wherein sesame hydrolysed protein and soya hydrolysed protein are embedded in liposomes. In a more particular embodiment, the liposomes comprise lecithin, which is a mixture of glycerophospholipids including phosphatidylcholine, phosphatidylethanolamine, phosphatidylinositol, and phosphatidic acid. Lecithin is thus used as a proliposome that helps sesame and soya hydrolysed proteins to penetrate in epidermis.

In a particular embodiment, the skin tightening composition (or composition capable of skin tightening) is in a form selected from the group consisting of solutions, aerosols and non-aerosol sprays, mousses, lotions, gels, sticks, ointments, pastes, creams, or gels. In a more particular embodiment it is a cream. Topical compositions used in the first aspect of the present invention can be prepared according to methods well known in the state of the art. The appropriate excipients and/or carriers, and their amounts, can readily be determined by those skilled in the art according to the type of formulation being prepared.

In another particular embodiment of the first aspect, the skin tightening composition is in form of a cream and it comprises:
- A composition comprising sesame hydrolysed protein, in particular said composition from 5.0 % w/w to 10.0 % w/w;
- A composition comprising hydroxyproline and organic silicium, in particular said composition from 3.0 % w/w to 6.0 % w/w;
- A composition comprising soy hydrolysed protein, in particular said composition from 3.0 % w/w to 6.0 % w/w;
- Lecithin, in particular from 0.10 % w/w to 0.20 % w/w; and
- Water to 100 % w/w of the composition.

The composition comprising hydroxyproline and organic silicium is also known as hydroprolylsilane. Hydroprolylsilane belongs to the chemical family of silanols. Silanols are hydrosoluble derivatives of organic silicon, obtained by condensation of methylsilanol, an organosilane, with numerous silanols functions, on a specific radical which confers to the silanol obtained, its specific action mode. Hydroprolylsilane is thus organis cilicium estabilized with hydroxyproline, and resulting from the reaction of methylsilanetriol with hydroxyproline, obtained by biotechnology. Any comercial hydroprolylsilane approved to b eused in cosmetics can be used. Particular comercial ones inlcude Hydroxyprolylsilane CN ®

In the same way, compositions comprising sesame and soy hydrolysate proteins are, in particular, composition comprising protein hydrolysates of different parts of the plant and, they are accompanied by other ingredients such as glycerine, copolymers, cellulose and water. Examples of commercially available sesame and soy protein hydrolysates containing compositions usable in the skin tightening composition include Sesaflash™ and Raffermine®. The skilled man will understood what is understood for commercially available protein hydrolysates of both plants and for cosmetically application.

Summarizing, a particular embodiment of the first aspect is a cosmetic method for ameliorating the unaesthetic appearance of skin caused by a condition selected from skin flaccidity, a lipodystrophy and a combination thereof, the method comprising:
a) topically administering a cosmetic skin tightening composition comprising a cosmetically effective amount of a sesame hydrolysed protein, a soy hydrolysed protein, organic silicium and hydroxyproline together with cosmetically acceptable excipients or carriers;
b) administering at different depths of anatomic structure defined by the skin and the subcutaneous tissue two cosmetic compositions capable of promoting collagen synthesis, wherein:
   one of the cosmetic compositions capable of promoting collagen synthesis is for subcutaneous administration and comprises a cosmetically effective amount of amino acids, one or more antioxidant compounds, and optionally one or more peptides from 3 to 50 amino acids length, together with cosmetically acceptable excipients or carriers; and
   the other cosmetic composition capable of promoting collagen synthesis is for intradermal administration and comprises a cosmetically effective amount of one or more co-factors for the enzymatic synthesis of collagen by fibroblasts cells, one or more antioxidant compounds, and optionally one or more peptides from 3 to 50 amino acids length, together with cosmetically acceptable excipients or carriers.

Particularities of any of the administered compositions have been disclosed above.

According to the first aspect, unaesthetic conditions of skin are ameliorated, said conditions caused by several causes in which subcutaneous fat is accumulated irregularly while or when connective tissue of hypodermis, dermis and epidermis is impaired. In a particular embodiment, the cosmetic method of the first aspect of the invention is for ameliorating the unaesthetic appearance of skin caused by a lipodystrophy selected from the group consisting of cellulite, local adiposities and combinations thereof. In yet a more particular embodiment is for ameliorating the unaesthetic appearance of skin caused by cellulite.

Since localized adiposities, as well as cellulite are common conditions in obese subjects, the method of the first aspect of the invention is also for ameliorating these conditions in particular in obese subjects.

In another particular embodiment of the first aspect of the invention, the condition causing unaesthetic appearance of the skin is caused by skin flaccidity.

In yet another particular embodiment is a combination of skin flaccidity and cellulite.

In another particular embodiment of the first aspect, the cosmetic method is performed in such a way that the administration of the cosmetic skin tightening composition in combination with the two cosmetic compositions capable of promoting collagen synthesis is carried out in more than one session with a resting time of at least one week between two consecutive sessions.

As will be illustrated in the examples, the cosmetic method of the invention provides good results in terms of increasing the skin elasticity when tested by means of a technology that applies negative pressure to a skin area and measures several parameters, in particular skin deformation and the time to return to initial state after deformation. In a particular embodiment, the method is further combined with another cosmetic treatment of the conditions selected from skin flaccidity, a lipodystrophy and a combination thereof. Particular examples of these other cosmetic methods are selected from the group selected of lymph drain messages, radiation application, in particular laser light, radiofrequency, infrared light, negative tissue massage, and combinations thereof.

With the aim of putting the cosmetic method into practice in an easy way, inventors have also developed kits with particular compositions. Thus, in a particular embodiment of the second aspect of the invention, the two cosmetic compositions capable of promoting collagen synthesis and for being administered at different depths of skin structure are injectable compositions, and the optional means for intradermal and subcutaneous administration comprise needles and syringes.

In another particular embodiment of the kit, optionally in combination with any embodiment above or below, the kit comprises a topical skin tightening composition as defined in any of the embodiments of the first aspect of the invention; an intradermal composition as defined in any of the embodiments of the first aspect of the invention; and a subcutaneous composition as defined in any of the embodiments of the first aspect of the invention.

Particular topical compositions as well as intradermal and subcutaneous compositions are illustrated in the examples below. All of them are, in a particular embodiment, comprised in the kit according to the invention.

As above indicated, many topical tightening compositions could be employed in the cosmetic method of the invention, since it is the combination with the administration with the other two compositions capable of promoting collagen synthesis and to be administered at different depths that surprisingly give the effect of ameliorating skin appearance. Nonetheless, particular new skin tightening compositions with sesame and soya hydrolysed proteins and organic silicium and hydroxyproline (i.e. hydroxyprolylsilane) have been developed for acting synergistically with the other two compositions.

Thus, in a particular embodiment of the third aspect of the invention, the topical skin tightening composition comprises a sesame hydrolysed protein, a soya hydrolysed protein, organic silicium and hydroxyproline. In a particular embodiment of the third aspect, the sesame hydrolysed protein and soya hydrolysed protein are embedded in liposomes. In a more particular embodiment, the liposomes comprise lecithin, which is a mixture of glycerophospholipids including phosphatidylcholine, phosphatidylethanolamine, phosphatidylinositol, and phosphatidic acid. Lecithin is thus used as a proliposome that helps sesame and soya proteins to penetrate in epidermis.

There have been indicated above, examples of commercial sesame and soy hydrolysate protein containing compositions.

In a more particular embodiment of the third aspect of the invention the skin tightening composition comprises in percentage by weight in relation with the total weight of the composition, from 5.0 to 10 % w/w of a composition comprising sesame hydrolysed protein; from 3.0 to 6.0 % w/w of organic silicium with hydroxyprolyne; from 3.0 to 6.0 % w/w of a composition comprising soy hydrolysed protein; from 0.10 % to 0.20 % w/w of lecithin and the balance of cosmetically acceptable excipients and/or carriers to reach 100 % w/w. In particular the cosmetically acceptable carrier is water.

In a particular embodiment of the cosmetic composition for subcutaneous administration comprising cosmetically effective amounts of amino acids, and an antioxidant compound, according to the fourth aspect, the composition comprises hyaluronic acid or a cosmetically acceptable salt thereof, nucleobases selected from thiamine, adenine and combinations thereof, amino acids selected from alanine, glycine, proline, leucine, lysine and combinations thereof, vitamin B, lipoic acid or a cosmetically acceptable salt thereof, and one or more oligopeptides.

In yet a more particular embodiment of the cosmetic composition for subcutaneous administration, it comprises hyaluronic acid or a cosmetically acceptable salt thereof, thiamine, alanine, cyanocobalamin, glycine, lipoic acid or a cosmetically acceptable salt thereof, proline, adenine, leucine, lysine, and one or more oligopeptides. Nucleobases and amino acids in this composition for subcutaneous administration are in form of the free acid or amine, or in form of a cosmetically acceptable salt thereof. In yet a more particular embodiment, the new cosmetic composition for subcutaneous administration comprises in percentage by weight in relation with the total weight of the composition an amount of hyaluronic acid or a cosmetically acceptable salt thereof from 0.05 % w/w to 0.5 %w/w; thiamine from 0.05 % w/w to 0.5 % w/w; alanine from 0.2 % w/w to 2.0 % w/w; cyanocobalamin (as synthetic form of vitamin B) from 0.001% to 0.2 % w/w; glycine from 1.0 % w/w to 5.0 % w/w; lipoic acid, or a cosmetically acceptable salt thereof from 0.005 % to 0.05 % w/w; proline from 0.1 % w/w to 1.0 % w/w; adenine from 0.005 % w/w to 0.2 % w/w; leucine from 0.1 % w/w to 0.5 % w/w; lysine from 0.05 % w/w to 0.3 % w/w; peptides from 1x10⁻⁴ % w/w to 5 x 10⁻⁴ % w/w; and the balance of cosmetically acceptable excipients and/or carriers to reach 100 % w/w. In particular the cosmetically acceptable carrier is water for injectables.

In a more particular embodiment of the fourth aspect, the cosmetic composition for subcutaneous administration comprises:
- Hyaluronic acid or a salt thereof, in particular from 0.05 % to 0.5 % w/w, more in particular 0.5 % w/w;
- Thiamine, in particular from 0.05 % to 0.5 % w/w, more in particular 0.1 % w/w;
- Alanine, in particular from 0.2 % to 2.0 % w/w, more in particular 0.8 % w/w
- Cyanocobalamine, in particular from 0.001 % to 0.2 % w/w, more in particular 0.05 % w/w;
- Glycine, in particular from 1.0 % to 5.0 % w/w, more in particular 1.0 % w/w;
- Lipoic acid or a salt thereof, in particular from 0.005 % to 0.05 % w/w, more in particular 0.02 % w/w;
- Proline, in particular from 0.1 % to 1.0 % w/w, more in particular 0.5 % w/w;
- Adenine, in particular from 0.005 % to 0.2 % w/w, more in particular 0.01 % w/w;
- Leucine, in particular from 0.1 % to 0.5 % w/w, more in particular 0.2 % w/w;
- Lysine, in particular from 0.05 % to 0.3 % w/w, more in particular 0.1 % w/w;
- Oligopeptide-24, in particular from 1x10⁻⁴ % w/w to 5 x 10⁻⁴ % w/w, more in particular 3x10⁻⁴ % w/w;
- Decapeptide-4, in particular from 1x10⁻⁴ % w/w to 5 x 10⁻⁴ % w/w, more in particular 2x10⁻⁴; and
- Water, amount to get 100% by weight of the composition.

New cosmetic compositions for intradermal administration have been developed, which are of particular application in the cosmetic method of the invention. Thus, according to a fifth aspect, the invention relates to compositions for intradermal administration comprising cosmetically effective amounts of one or more co-factors for the enzymatic synthesis of collagen by fibroblast cells, and one or more antioxidant compounds, together with cosmetically acceptable excipients and/or carriers.

In a particular embodiment of these new cosmetic compositions for intradermal administration, it comprises ascorbic acid or a cosmetically acceptable salt thereof, , in particular a salt of an alkali metal, more in particular sodium salt; lactic acid or a salt thereof, in particular a salt of an alkali metal, more in particular sodium salt; DMAE; a dicarboxylic acid salt of zinc; dicarboxylic acid salt of copper, more in particular gluconate salts of zinc and copper; chondroitin sulphate; and a peptide.

In yet a more particular embodiment, the percentage by weight in relation with the total weight of the composition of the above listed compounds is as follows: from 0.1 % w/w to 10 % w/w of ascorbic acid or a cosmetically acceptable salt thereof; from 2.0 % w/w to 5.0 % w/w of lactic acid or a salt thereof; from 1.0 % w/w 20.0 % w/w of DMAE; from 0.02 % to 0.2 % w/w of zinc gluconate; from 0.1 % to 0.5 % w/w of copper gluconate; from 0.1 % to 0.5 % w/w of chondroitin sulphate; and from 1x10⁻⁴ % w/w to 5 x 10⁻⁴ % w/w of one or more peptides from 3 to 50 amino acids length.

In a more particular embodiment of the fifth aspect of the invention, the intradermal administered composition comprises
- Ascorbic acid, in particular from 0.1 % to 10 % w/w, more in particular of 5.0 % w/w;
- Sodium lactate, in particular from 2.0 % to 5.0 % w/w, more in particular of 1.0 % w/w;
- DMAE, in particular from 1.0 % w/w 20.0 % % w/w, more in particular of 2.0 % w/w
- Zinc gluconate, in particular from 0.02 % to 0.2 % w/w, more in particular of 0.1 % w/w;
- Chondroitin sulphate, in particular from 0.1 % to 0.5 % w/w, more in particular of 0.1 % w/w;
- Copper gluconate, in particular from 0.01 % to 0.05 % w/w, more in particular of 0.025 % w/w
- Tripeptide-6, in particular from 1x10⁻⁴ % w/w to 5 x 10⁻⁴ % w/w, more in particular 1x10⁻⁴ % w/w; and
- Water, amount to get 100% by weight of the composition.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### Example 1. Cosmetic composition for subcutaneous administration

The following composition was prepared using water for injectables (wfi) as carrier. This composition was proposed and tested as composition for subcutaneous administration:

| Ingredient | w/w % en composition | (mg/ml) | range (mg/ml) |
|---|---|---|---|
| Hyaluronic acid | 0,5000% | 5 | 0,5-5 |
| Thiamine | 0,1000% | 1 | 0,5-5 |
| Alanine | 0,8000% | 8 | 2-20 |
| Cyanocobalamine | 0,0500% | 0,5 | 0,01-2 |
| Glycine | 1,0000% | 10 | 10-50 |
| Lipoic acid | 0,0200% | 0,2 | 0,05-0,5 |
| Proline | 0,5000% | 5 | 1-10 |
| Adenine | 0,0100% | 0,1 | 0,05-2 |
| Leucine | 0,2000% | 2 | 1-5 |
| Lysine | 0,1000% | 1 | 0,5-3 |
| Oligopeptide-24⁽¹⁾ (INCI name) | 0,0003% | 0,003 | 0,001-0,005 |
| Decapeptide-4⁽²⁾ (INCI name) | 0,0002% | 0,002 | 0,001-0,005 |
| wfi | q.s 100 % | | |

| | | | |
|---|---|---|---|
| ⁽¹⁾Oligopeptide-24 is the synthetic peptide containing 13 amino acids consisting of arginine, aspartic acid, cysteine, isoleucine, glutamic acid, glycine, methionine, and tyrosine (also referred as so according to IUPAC). ⁽²⁾Decapeptide-4 is a synthetic peptide consisting of arginine, aspartic acid, cysteine, glutamic acid, leucine, methionine and tyrosine (also referred as so according to IUPAC) | | | |

This composition was prepared by weighing the amounts of each of the ingredients and adding to a tank with wfi and with agitating means, one by one of the ingredients until complete homogenization. The final mixture was then filtrated to avoid any bacterial charge. The filtrated mixture was filled in 10 ml vials and the vials were sterilized by autoclave (8 minutes in sterilization tank at 121 °C).

### Example 2. Cosmetic composition for intradermal administration

The following composition was prepared using water for injectables (wfi) as carrier. This composition was proposed and tested as composition for intradermal administration:

| Ingredient | w/w % en composition | (mg/ml) | range (mg/ml) |
|---|---|---|---|
| Ascorbic acid | 5,0000% | 50 | 10-100 |
| Sodium lactate | 1,0000% | 10 | 10-20 |
| DMAE | 2,0000% | 20 | 1-20 |
| Zinc Gluconate | 0,1000% | 1 | 0,2-2 |
| Chondroitin Sulphate | 0,1000% | 2,5 | 1-5 |
| Copper Gluconate | 0,0250% | 0,25 | 0,1-0,5 |
| Tripeptide-6* (INCI name) | 0,0001% | 0,001 | 0,001-0,005 |
| wfi | q.s 100 % | | |

| | | | |
|---|---|---|---|
| *Tripeptide-6 is a synthetic peptide consisting of glycine, hydroxyproline and proline. | | | |

This composition was prepared by weighing the amounts of each of the ingredients and adding to a tank with wfi and with agitating means, one by one of the ingredient until complete homogenization. The final mixture was then filtrated to avoid any bacterial charge. The filtrated mixture was filled in 10 ml vials and the vials were sterilized by autoclave (8 minutes in sterilization tank at 121 °C).

### Example 3. Topical skin tightening composition

The following composition was prepared using water as carrier. This composition was proposed and tested as topical skin tightening composition

| Ingredient | % w/w in formula | Recommended dose range. |
|---|---|---|
| Sesaflash ™ | 5,00% | 5-10% |
| Hydroxyprolisilane CN ® | 3,00% | 3-6% |
| Raffermine ® | 2,00% | 3-6% |
| Lecithin | 0,10% | |
| Cosmetically aceptable excipients and carriers (water, emollient (Cetiol®), perfume, thickening polymer (Sepimax Zen and Sepiplus 400), emulsifier (Easynov), preservative (Euxyl K700), deodorant-emollient (Sensiva sc50), sodium citrate, citric acid, sodium EDTA, glycerin) | q.s. 100% | |

Particular compositions of the ingredients are listed below:

| | | | |
|---|---|---|---|
| Sesaflash ™ | glycerin | 35,0000% | 5,0000% |
| | acrylates copolymer | 17,3000% | |
| | vp/polycarbamyl polyglycol ester | 0,5000% | |
| | INCl name: hydrolyzed sesame protein pg-propyl methylsilanediol (proteins from sesame hydrolysed, and 2-hydroxy-3-(3-(methyldihydroxysilyl)propoxy)propyl derivatives) | 0,4000% | |
| | Water | 46,8000% | |
| Raffermine ® | Water | 97,0000% | 2,0000% |
| | hydrolyzed soy flour comprising glycoproteins and structural polysaccharides (cellulose)/INCI name: Hydrolyzed Soy Flour/CAS N°:9010-10-0 | 1,5000% | |
| | phenoxyethanol | 1,5000% | |
| Lecithin | lecithin | 100,0000% | 0,1000% |
| Hydroxyprolisilane CN ® | methylsilanol hydroxyproline aspartate | 79,5000% | 3,0000% |
| | butylene glycol | 20,0000% | |
| | sodium benzoate | 0,5000% | |

Although particular commercially available compositions comprising soy and sesame protein hydrolysates were used, other with similar compositions could be employed.

This skin tightening composition was prepared by weighing and heating to 65 °C-70 °C the emollients, perfume, emulsifier and thickening polymer (Sepimax) (Mixture A). Weighing separately water, the preservative, the deodorant-emollient, sodium citrate, citric acid, sodium EDTA, and glycerine and heating to 65 °C-70 °C (Mixture B). Mixtures A and B were joined and emulsified at 70 °C and let to cold to 40 °C. Then Sesaflash™, Hydroxyprolisilane CN ® and Raffermine ® were individually added until complete homogeneity. A mixture of lecithin and water was added. And finally, the thickening polymer Sepiplus 400 was added. This skin tightening composition had a cream texture.

### Example 4. In vivo assay. Test of skin flaccidity.

The compositions of Examples 1, 2 and 3 were tested in several patients (39 women with skin flaccidity in abdomen and arms)

Application of the subcutaneous composition was performed by injecting at 6-7 mm in relation with the surface of skin a set of 9 injections of 0.5 cm³ in a skin area of 10 cm x 15 cm, each injection spotted at 1 cm approximately of distance.

Next, the intradermal composition was applied in a depth from 3 to 4 mm in relation with the surface of skin. 50-60 injections of 0.1 cm³ and also with a distance between injections of 1 cm approximately were applied in the same skin area.

Finally, the cream of example 3 was applied to the previously injected area.

This protocol was repeated with a schedule of three sessions (with application of the three compositions) per month.

In order to evaluate skin elasticity as a demonstration of the quality of the collagen, elastin and other extracellular matrix proteins, the Cutometer technology was employed. The Cutometer® is destined to measure elasticity of the upper skin layer using negative pressure which deforms the skin mechanically. The measuring principle is based on the suction method. Negative pressure is created in the device and the skin is drawn into the aperture of the probe and after a defined time released again. Inside the probe, the penetration depth is determined by a non-contact optical measuring system. This optical measuring system consists of a light source and a light receptor, as well as two prisms facing each other, which project the light from transmitter to receptor. The light intensity varies due to the penetration depth of the skin. The resistance of the skin to the negative pressure (firmness) and its ability to return into its original position (elasticity) are displayed as curves (penetration depth in mm/time) in real time during the measurement. This measurement principle allows getting information about the elastic and mechanical properties of skin surface and enables to objectively quantify, among other properties of skin, skin aging and skin flaccidity. From these curves interesting measurement parameters can be calculated.

A Cutometer® dual MPA 580 (Courage Khazaka, GMbH) was used prior to the application of the three compositions and after the whole session (application of the three compositions as above indicated) according to the method of the invention.

Next Table 1 shows an objective evaluation of the method. PSKey shows the Patient satisfaction assessment, from 1(low satisfaction) to 5 (high satisfaction). DSKey shows the Technician/Medical satisfaction assessment, from 1(low satisfaction) to 5 (high satisfaction). In addition, VAS is the Virtual analogue scale for pain self-assessment for each of the vials, from 0 (low pain) to 5 (high pain). Table 1 depicts the mean of all 39 patients.

**Table 1. Subjective evaluation of the method**

| PSKey | DSKey | VAS | | |
|---|---|---|---|---|
| | | Vial 1 (s.c) | Vial 2 (i.d) | Vial 3 (topical) |
| 4.26 | 4.05 | 2.33 | 2.77 | 0 |

Data from Cutometer® are listed in Table 2, wherein several parameters related with skin elasticity are shown. In particular there are shown the R-parameters R0, R2, R5 and R7 derived from Cutometer®. R0 represents the passive behaviour of the skin when force is applied to it. It is the first maximal amplitude, the highest point of the first curve and finds correlation with skin firmness. R2 represents the portion of the curve between the maximal amplitude and the skin re-deformation capability. This is the elasticity of the skin and the closer the value gets to 1 the more elastic the tissue is. R5 correlates with elasticity, but solely analyzes the elastic component of the viscoelastic curve derived from Cutometer®, not anchoring it to the viscous component of said viscoelastic curve. R7 represents the relation between the elastic portion of the elasticity curve and the complete (viscoelastic) curve: the closer the value is to 1 (100 %) the more elastic the curve (and so the skin) is. Again Table 2 shows the mean of the values of all subjects for each of the parameters. *RX* means the value of the parameter at t=0 (also indicated as RX(0)), which was before the performance of the cosmetic method. *RX post* is the value of the parameter one month after application of the method, thus 2 months after determination of *RX(0).*

**Table 2. Objective evaluation of the efficacy of the method.**

| Cutometry parameters with method of the invention | | | | | | | |
|---|---|---|---|---|---|---|---|
| R0 | R0 post | R2 | R2 post | R5 | R5 post | R7 | R7 post |
| 0.28 | 0.34 | 0.65 | 0.76 | 0.42 | 0.48 | 0.28 | 0.33 |
| Increase *RX post* vs *RX(0)* | 22.90% | Increase | 16.82 % | Increase | 14.40 % | Increase | 20.73 % |

| Cutometry parameters with a comparative method (*) | | | | | | | |
|---|---|---|---|---|---|---|---|
| R0 | R0 post | R2 | R2 post | R5 | R5 post | R7 | R7 post |
| Increase *RX post* vs *RX(0)* | 10.38% | Increase | 16.59 % | Increase | 11.21 % | Increase | 16.16 % |

Comparative method (*) consisted in the application of an autologous antiaging serum (AAS), prepared from activated blood with the contact of borosilicate medical grade glass pebbles and centrifugation. This AAS was applied in malar region by several low-volume intra-dermal injections and one single high-volume intramuscular injection. (See Pinto et al. Study to evaluate the aesthetic clinical impact of an autologous antiaging serum. J Drugs Dermatol; 2013 Mar;12(3):322-6).

All these data allow concluding that the method of the invention was well-tolerated and accepted by the 39 women. In addition, it was highly effective in terms of improving skin elasticity and firmness, which is an indirect measure of skin flaccidity. This improvement was even higher than the one associated to another method, in which an autologous serum was injected at different levels of anatomical structure also with the aim of improving elasticity of skin tissue.

### Citation List

### Patent Literature

- WO2013093947
- EP1928466
- EP0857480
- EP2263683

### Non Patent Literature

- Alster et al., "Treatment of cellulite with optical devices: an overview with practical considerations", Lasers in Surgery and Medicine 2006, vol. 38, pp:727-730.
- Pinto et al. "Study to evaluate the aesthetic clinical impact of an autologous antiaging serum", J Drugs Dermatol 2013, vol.12(3), pp:322-6

## Claims

1. A cosmetic method for ameliorating the unaesthetic appearance of skin caused by a condition selected from skin flaccidity, a lipodystrophy and a combination thereof, the method comprising topically administering a cosmetic composition capable of skin tightening in combination with two cosmetic compositions capable of promoting collagen synthesis, said compositions capable of promoting collagen synthesis being administered at different depths of anatomic structure defined by the skin and the subcutaneous tissue.

2. The cosmetic method according to claim 1, wherein one composition capable of promoting the collagen synthesis is subcutaneously administered; and one composition capable of promoting the collagen synthesis is intradermally administered.

3. The cosmetic method according to any one of claims 1-2, wherein one composition capable of promoting the collagen synthesis is subcutaneously administered and the subcutaneously administered composition comprises a cosmetically effective amount of amino acids and one or more antioxidant compounds together with cosmetically acceptable excipients or carriers.

4. The cosmetic method according to any one of claims 2-3, wherein the subcutaneously administered composition comprises amino acids selected from the group consisting of alanine, glycine, proline, leucine, lysine and combinations thereof.

5. The cosmetic method according to any one of claims 1-4, wherein one composition capable of promoting the collagen synthesis is intradermally administered, and the intradermally administered composition comprises a cosmetically effective amount of one or more co-factors for the enzymatic synthesis of collagen and one or more antioxidant compounds, together with cosmetically acceptable excipients or carriers.

6. The cosmetic method according to any one of claims 1-5, wherein the cosmetic composition capable of skin tightening comprises a cosmetically effective amount of a sesame hydrolysed protein, a soya hydrolysed protein, organic silicium and hydroxyproline, together with cosmetically acceptable excipients or carriers.

7. The cosmetic method according to any one of claims 1-6, wherein the lipodystrophy is selected from the group consisting of cellulite, local adiposities and combinations thereof.

8. The cosmetic method according to any one of claims 1-7, wherein the administration of the cosmetic composition capable of skin tightening in combination with the two cosmetic compositions capable of promoting collagen synthesis is carried out in more than one session with a resting time of at least one week between two consecutive sessions.

9. The cosmetic method according to any one of claims 1-8, further comprising another cosmetic treatment of the conditions selected from skin flaccidity, a lipodistrophy and a combination thereof.

10. A kit for ameliorating the unaesthetic appearance of skin caused by a condition selected from skin flaccidity, a lipodystrophy and a combination thereof, said kit comprising:
- a topical skin tightening composition;
- two skin cosmetic compositions capable of promoting collagen synthesis and for being administered at different depths of the anatomic structure defined by the skin and the subcutaneous tissue;
- optionally, means for intradermal and subcutaneous administration of the compositions capable of promoting collagen synthesis compositions; and
- optionally, use instructions.

11. Topical skin tightening composition comprising a cosmetically effective amount of a sesame hydrolysed protein, a soy hydrolysed protein, organic silicium and hydroxyproline, together with cosmetically acceptable excipients or carriers.

12. Cosmetic composition for subcutaneous administration comprising a cosmetically effective amount of amino acids, one or more antioxidant compounds, wherein one of the antioxidant compounds is lipoic acid, together with cosmetically acceptable excipients or carriers.

13. Cosmetic composition for intradermal administration comprising a cosmetically effective amount of one or more co-factors for the enzymatic synthesis of collagen by fibroblasts cells and one or more antioxidant compounds, together with cosmetically acceptable excipients or carriers.
